(19) Europäisches Patentamt
European Patent Office
Office européen des brevets

(11) **EP 2 564 771 A1**

(12) **EUROPÄISCHE PATENTANMELDUNG**

(43) Veröffentlichungstag:
06.03.2013 Patentblatt 2013/10

(51) Int Cl.:
*A61B 5/0402* (2006.01)  *A61B 5/06* (2006.01)

(21) Anmeldenummer: 11075206.0

(22) Anmeldetag: 05.09.2011

(84) Benannte Vertragsstaaten:
AL AT BE BG CH CY CZ DE DK EE ES FI FR GB
GR HR HU IE IS IT LI LT LU LV MC MK MT NL NO
PL PT RO RS SE SI SK SM TR
Benannte Erstreckungsstaaten:
BA ME

(71) Anmelder: ECP Entwicklungsgesellschaft mbH
12247 Berlin (DE)

(72) Erfinder: Ferrari, Markus
07747 Jena (DE)

(74) Vertreter: Pfenning, Meinig & Partner GbR
Patent- und Rechtsanwälte
Joachimstaler Strasse 12
10719 Berlin (DE)

(54) **Medizinprodukt mit einem Funktionselement zum invasiven Einsatz im Körper eines Patienten**

(57) Um beim invasiven Einsatz eines Funktionselementes (5) im Körper eines Patienten eine möglichst genaue Lagebestimmung dieses Funktionselementes ohne den Einsatz bildgebender Verfahren durchführen zu können, ist das Funktionselement mit einem Hauptsensor (6) verbunden, der beispielsweise elektrophysiologische Signale des Herzens des Patienten aufnimmt, die mit anderen, über mehrere an der Körperoberfläche verteilte Sensoren (7, 8, 9, 10, 11) aufgenommenen elektrophysiologischen Herzsignalen abgeglichen werden, um durch die Verknüpfung die Lagebestimmung des Funktionselementes zu ermöglichen.

Fig. 3

EP 2 564 771 A1

**Beschreibung**

Die Erfindung liegt auf dem Gebiet der Medizintechnik, insbesondere der Mikromechanik und der medizinischen Messtechnik.

In der modernen Medizin werden vielfältig und in zunehmendem Maße mikroinvasive Techniken eingesetzt, um bei minimaler Belastung des Patienten einen maximalen unterstützenden, therapeutischen oder diagnostischen Erfolg zu erzielen.

Beispiele für solche Techniken sind die Einführung von Stents in Blutgefäße, der Einsatz von Thrombenfiltern, die Beseitigung von Belägen in Blutgefäßen durch Fräsen sowie die Unterstützung oder ein temporärer oder anteiliger Ersatz der Herzfunktion eines Patienten durch in Blutgefäße eingeführte Mikropumpen.

Für viele dieser Techniken ist es notwendig, mittels eines Katheters entsprechende Funktionselemente über eine Blutbahn in den Körper des Patienten einzuführen und diese innerhalb der Blutbahn möglichst sinnvoll zu positionieren.

Eine möglichst genaue Lagekontrolle ist dabei sowohl anfänglich oder während eines akuten Einsatzes, wie beispielsweise bei einem Fräskopf, als auch langfristig nicht nur wünschenswert, sondern für den Erfolg der invasiven Maßnahme mit entscheidend.

Oft erfolgt eine Lageuntersuchung der entsprechenden Funktionselemente unter Röntgendurchleuchtungskontrolle oder mittels transösophagealer Echokardiographie. Diese Methoden sind jedoch an aufwendige und nicht immer verfügbare Vorrichtungen gebunden. Dies macht die Positionsbestimmung nicht nur aufwendig und teuer, sondern ist in vielen Fällen wegen hohen Zeitdrucks nicht praktikabel oder nicht sinnvoll. Unter Notfallbedingungen kann zwar beispielsweise eine intraaortale Pumpe auch blind gelegt werden, indem der Abstand zwischen der Punktionsstelle in der Leiste und der Jugulargrube ausgemessen wird. Dies setzt jedoch normale anatomische Gegebenheiten voraus und birgt die Gefahr einer Fehlplatzierung in die kontralaterale Leistenschlagader. Auch ist diese Methode wegen der nicht vorhersagbaren individuellen anatomischen Prägung der Hauptschlagader unpräzise. Kann das Funktionselement, insbesondere die Pumpe, hierdurch nicht optimal platziert werden, so ist die Unterstützungsleistung im Falle einer Pumpe nicht optimal, und andere Funktionselemente können ebenfalls nicht optimal funktionieren.

Aus dem Stand der Technik sind außer der reinen Durchleuchtungsmethode noch andere Möglichkeiten zu einer mehr oder weniger genauen Positionsbestimmung eines Bauelementes, insbesondere eines Herzkatheters, bekannt.

So offenbart die US-Patentschrift 5 983 126 die Anwendung dreier orthogonal zueinander ausgerichteter externer Signale außerhalb des Patientenkörpers auf das Positionierungsgebiet, wobei mittels einer Sonde an dem Funktionselement der Einfluss der drei Signale gemessen und daraus auf die Position geschlossen werden kann. Die entsprechenden externen Signale müssen so beschaffen sein, dass sie nicht mit den elektrophysiologischen Signalen des Herzens interferieren.

Die US 6 226 546 B1 beschreibt ein Verfahren zur Lokalisierung eines Katheters mit einer Anzahl von akustischen Sonden am Kopf des Katheters, wobei über akustische Empfänger von den Sonden ausgesandte Signale empfangen werden, die durch eine Verarbeitungseinrichtung zur Positionsbestimmung und zur Abbildung des anatomischen Umfeldes der Sonden verarbeitet werden.

Aus der US 5 391 199 ist eine Methode zur Lokalisierung eines Katheters mit einer Genauigkeit von etwa 1 mm bekannt, bei der ein Sender außerhalb des Patientenkörpers mittels Antenne Signale abstrahlt und an der Spitze des Katheters Empfangsantennen vorgesehen sind, die mit einem Empfänger zur Verarbeitung der Signale verbunden sind. Die Signale werden mit entsprechenden Referenzsignalen von Referenzantennen im Körper des Patienten verglichen. Aus der US 6 892 091 B1 sind Geräte und Verfahren zur Aufnahme und Abbildung der elektrophysiologischen Aktivität innerhalb eines Herzens mittels eines Herzkatheters bekannt. Der entsprechende Katheter verfügt auch über einen Positionssensor, der als elektromagnetischer Sensor ausgebildet ist. Als Ausführungsbeispiel für den Einsatz eines derartigen Positionssensors ist dort die Anwendung von außerhalb des Patientenkörpers erzeugten Magnetfeldern beschrieben, die auf den Positionssensor wirken, so dass mittels des Sensors sowohl eine Lage als auch eine Orientierung des Sensors und somit des Katheters bestimmt werden kann. Es ist zudem der Vergleich mit Referenzsignalen von zusätzlich eingebrachten Kathetern beschrieben.

Den aus dem Stand der Technik bekannten Verfahren ist ein hoher apparativer Aufwand entweder in Form von bildgebenden Einrichtungen oder von zusätzlichen Signalquellen außerhalb des Patientenkörpers gemeinsam.

Die vorliegende Erfindung hat sich die Aufgabe gestellt, ein Medizinprodukt zu schaffen, das eine einfache und zuverlässige Lagebestimmung beim invasiven Einsatz mit besonders geringem apparativem Aufwand erlaubt.

Die Aufgabe wird gemäß der Erfindung mit den Merkmalen des Patentanspruchs 1, 11 oder 12 gelöst.

Dabei ist in der Variante des Patentanspruchs 1 ein Medizinprodukt mit einem Funktionselement zum invasiven Einsatz im Körper eines Patienten mit einem Hauptsensor vorgesehen, der in einem festen räumlichen Bezug zu dem Funktionselement steht. Insbesondere hat der Hauptsensor einen festen, bekannten Abstand von dem Funktionselement. Zudem ist eine Verarbeitungseinrichtung vorgesehen, die Signale des Hauptsensors empfängt, welche die von dem Hauptsensor erfassten Signale oder Messwerte repräsentieren, und die wenigstens eine, die Position des Funktionselementes repräsentierende Größe aus Signalen des Hauptsensors unter Berücksichtigung elektrophysiologischer Signale des Herzens ermittelt.

Es ist seit langem bekannt, elektrophysiologische Signale an einem schlagenden Herzen zu messen. Eine typische Anwendung ist hierbei das übliche Elektrokardiogramm mit Elektroden, die je nach Art der Ableitung und der Komplexität in passender Anzahl auf die Haut eines Patienten aufgebracht sind.

Die zeitveränderlichen Signale werden üblicherweise zu einem Vektor verrechnet, der einen das jeweilige individuelle Herz charakterisierenden Fingerabdruck repräsentiert. Da die jeweils abgeleiteten elektrophysiologischen Signale stark von dem Ort der Ableitung abhängen, kann von einem Vergleich eines gemessenen Signals mit einem typischen Elektrokardiogramm derselben Person auf die Position der Messung/Ableitung rückgeschlossen werden. Ebenso kann gegebenenfalls auf die Position einzelner Ableitungselektroden geschlossen werden.

Insofern sieht die Erfindung eine entsprechende Verarbeitungseinrichtung vor, die entweder mit Elektroden direkt in Verbindung steht oder der entsprechende vorverarbeitete Daten von EKG-Elektroden zugeleitet werden können.

Werden die Signale, die mit dem Hauptsensor aufgenommen werden, kontinuierlich oder regelmäßig mit den übrigen elektrophysiologischen Daten des Herzens verglichen oder verknüpft, so kann eine laufende Lagekontrolle des Hauptsensors und damit auch des mit diesem in einem festen räumlichen Bezug stehenden Funktionselementes stattfinden. In besonders einfacher Ausprägung ist der Hauptsensor beispielsweise an dem Funktionselement selbst oder in festem Abstand zu diesem angeordnet, z. B. an einem seiner Enden, wenn das Funktionselement als im Wesentlichen länglicher Körper ausgebildet ist.

In analoger Weise kann das Verfahren auch mit Hilfe von Impedanzmessungen oder auch der Analyse einer Pulskurve im Vergleich mit einer peripheren Arterie (z.B. Arm- oder Beinarterie) angewendet werden. Hier wird der zeitliche Versatz zwischen den Druckanstiegsmaxima bzw. -minima gemessen und zeigt Änderungen der Lage oder des Kreislaufverhaltens an.

In einer besonderen Ausprägung der Erfindung ist die Verarbeitungseinrichtung direkt mit Hilfssensoren verbindbar, die entsprechende elektrophysiologische Herzsignale ableiten, und die Verarbeitungseinrichtung kann die Signale des Hauptsensors mit aktuell erfassten Signalen der Hilfssensoren oder mit hieraus ermittelten Daten, wenn diese beispielsweise zu einem EKG vorverarbeitet sind, weiterverarbeiten. Vorteil dieser Vorgehensweise ist beispielsweise, dass der zeitliche Rhythmus der durch den Hauptsensor erfassten Signale direkt mit dem Rhythmus der Signale der Hilfssensoren synchronisiert werden kann.

Es kann dann das über den Hauptsensor gewonnene Signal mit dem Vektor der Extremitätenableitungen beispielsweise im Verhältnis zu einer spezifischen Extremitätenableitung nach der von Goldberger beschriebenen Technik genutzt werden, um einen optimalen Lagevektor zu definieren. Dieser Vektor kann kontinuierlich mit dem über den Hauptsensor gewonnenen EKG-Vektor verglichen werden, so dass die tatsächliche Position des Hauptsensors und damit des Funktionselementes für jeden Schlag des Herzens ermittelt und mit einer Wunschposition verglichen werden kann. Daten über gemessene Vektoren und hierzu erfasste Daten des Hauptsensors können zur Kalibrierung bei verschiedenen bekannten Positionen des Hauptsensors und/oder des Funktionselementes aufgenommen werden (beispielsweise bei gleichzeitiger Anwendung eines bildgebenden Verfahrens), und auf diese Weise kann eine Art "Landkarte" der Positionen anhand von Beispieldaten gespeichert und später mit aktuell erfassten Daten verglichen werden. Es kann beispielsweise jeweils der dem aktuell erfassten Datensatz ähnlichste Datensatz ermittelt und aus den zu diesem ähnlichsten Datensatz gespeicherten Positionsdaten auf die aktuelle Position des Hauptsensors und/oder Funktionselementes geschlossen werden.

Insofern ist es auch möglich, dass die Verarbeitungseinrichtung mit einer Speichereinrichtung verbunden ist, in der früher erfasste elektrophysiologische Herzsignale, insbesondere des Herzens des Patienten, gespeichert sind, und dass die Verarbeitungseinrichtung zur Ermittlung der die Position des Funktionselementes repräsentierenden Größe Signale des Hauptsensors mit gespeicherten Herzsignalen und/oder anderen früher erfassten Daten oder hieraus ermittelten Daten verknüpft.

Es ist dann eine zeitliche Skalierung der aktuell mittels des Hauptsensors gewonnenen Daten zum Vergleich mit den gespeicherten EKG-Daten notwendig oder zumindest vorteilhaft, bevor der aktuell gewonnene Vektor bzw. die aktuell gewonnenen Daten mit den gespeicherten Daten verglichen werden können.

Es ist auch denkbar, die gespeicherten Referenz-EKG-Daten mit aktuell gewonnenen EKG-Daten der Hilfssensoren in Beziehung zu setzen und hieraus Daten zu gewinnen, mit denen die Signale des Hauptsensors verglichen werden.

Der Hauptsensor weist typischerweise eine Elektrode und/oder Antenne zur Aufnahme von elektrophysiologischen Signalen auf. Im Körper des Patienten pflanzen sich solche Signale einerseits elektrodynamisch, andererseits galvanisch fort, so dass sie auch im Abstand von dem Herzen gut aufgenommen werden können. Die Ankopplung einer entsprechenden Elektrode an einen Messkreis entscheidet im Wesentlichen darüber, welches Frequenzverhalten erzielt wird und ob es sich eher um eine Messelektrode oder eine Antenne handelt.

Vorteilhaft können auch mehr als eine Elektrode oder Antenne an dem Hauptsensor vorgesehen sein. Beispielsweise kann sich eine genauere Lagebestimmung und Orientierungsbestimmung des Hauptsensors mit zwei voneinander beabstandeten Elektroden/Antennen ergeben. Es sind jedoch auch mehr als zwei Elektroden denkbar. Es kann dann ein mehrdimensionaler Vektor aus den mit dem Hauptsensor erfassten Daten gewonnen werden.

Besonders vorteilhaft ist die Erfindung mit einem Funktionselement realisierbar, das mittels eines Katheters durch ein Blutgefäß bewegbar ist. Besonders interessant können Aussagen über Funktionselemente sein, die typischerweise außerhalb oder auch innerhalb des Herzens eingesetzt werden und bei denen die Entfernung vom Ventrikelein- oder -ausgang oder die Position entlang des Blutgefäßes für einen erfolgreichen Einsatz ausschlaggebend ist.

Typische Funktionselemente gemäß der Erfindung können beispielsweise Blutpumpen, insbesondere intraaortale Ballonpumpen, Rotationspumpen oder innerhalb von Blutgefäßen einsetzbare Fräsköpfe, Stents oder Thrombenfilter sein.

Insbesondere bei Blutpumpen ist die Positionierung in Bezug auf das Ventrikel besonders wichtig. Eine intraaortale Ballonpumpe beispielsweise muss außen vor dem Ventrikel in einem bestimmten Abstand positioniert werden, während eine Rotationspumpe teilweise in das Ventrikel hineinragen sollte.

Insofern kann gemäß der Erfindung vorgesehen sein, dass die die Position des Funktionselementes repräsentierende Größe der Abstand des Funktionselementes von der Mündung eines Blutgefäßes, in dem sich das Funktionselement befindet, in eine Herzkammer, insbesondere entlang dem Verlauf des Blutgefäßes gemessen, ist.

Gemäß einer besonderen Ausprägung der Erfindung kann auch vorgesehen sein, dass der Hauptsensor einen Sensor zur Erfassung einer strömungsmechanischen Größe, insbesondere einen Drucksensor, aufweist und dass die Verarbeitungseinrichtung das zeitliche Verhältnis zwischen aktuell erfassten elektrophysiologischen Herzsignalen und einer die Blutströmung an der Position des Hauptsensors charakterisierenden Messgröße erfasst und hieraus eine für die Position des Funktionselementes repräsentative Größe ermittelt. In diesem Fall wird im Wesentlichen die Laufzeit von durch die Herzaktivität erzeugten Druckänderungen im Blutkreislauf zur Bestimmung des Ortes des Hauptsensors bzw. des Funktionselementes benutzt. Da die elektrophysiologischen Signale praktisch verzögerungsfrei aufgenommen werden, die Wanderung einer Druckwelle jedoch wesentlich langsamer geschieht, kann der Zeitverzug beispielsweise eines Druckwellenmaximums bei bekannter Wanderungsgeschwindigkeit zur Berechnung des Abstands genutzt werden. Es können anstelle des Druckwellenmaximums jedoch auch andere typische Punkte des Druckverlaufs im Gefäßsystem verwendet werden.

Die Erfindung bezieht sich außer auf ein Medizinprodukt, wie dies oben teilweise beispielhaft beschrieben wurde, auch auf ein Verfahren zum Betrieb eines Funktionselementes im Körper eines Patienten, insbesondere in einem Blutgefäß, dadurch gekennzeichnet, dass eine die Position des Funktionselementes repräsentierende Größe dadurch ermittelt wird, dass ein in festem räumlichem Bezug zu dem Funktionselement stehender Hauptsensor wenigstens einen durch die Herzfunktion des Patienten bestimmten Parameter lokal erfasst und in Signale umwandelt und dass weitere die Herzfunktion charakterisierende Daten oder Signale mit den Signalen des Hauptsensors verknüpft werden.

In einer vorteilhaften Ausgestaltung der Erfindung kann vorgesehen sein, dass die Verarbeitungseinrichtung zur Verarbeitung von Signalen eines Impedanzsensors und/oder eines Blutdrucksensors und/oder eines Atmungsaktivitätssensors und/oder eines Sensors für den Sauerstoffgehalt im Blut eingerichtet ist und/oder dass die Verarbeitungseinrichtung mit einem oder mehreren Sensoren der genannten Sensorarten verbunden ist. Damit ist es möglich, über die Verknüpfung einer oder mehrerer der genannten Messgrößen mit erfassten Signalen oder Messergebnissen des Hauptsensors entweder direkt oder über einen Vergleich mit gespeicherten Referenzdaten dessen Position zu bestimmen. Der Hauptsensor kann ebenso zur Erfassung der genannten Messgrößen Impedanz, Blutdruck, Atmungsaktivität oder Sauerstoffgehalt geeignet sein.

Im Folgenden wird die Erfindung in einem Ausführungsbeispiel beschrieben und in einer Zeichnung gezeigt.

Dabei zeigt

Fig. 1 schematisch die Ansicht einer intraaortalen Ballonpumpe in einem Blutgefäß in Herznähe, wobei der Ballon seine komprimierte Form annimmt,

Fig. 2 die Konstellation aus Fig. 1 mit einem expandierten Ballon,

Fig. 3 schematisch den Oberkörper eines Patienten mit Elektroden zur Ableitung von EKG-Signalen sowie einer intraaortalen Ballon-pumpe mit einem Hauptsensor,

Fig. 4 schematisch ein Blutgefäß mit einem Funktionselement, einem Hauptsensor sowie Hilfssensoren und einer Verarbeitungs- einrichtung,

Fig. 5 schematisch eine alternative Verarbeitungseinrichtung,

Fig. 6 eine weitere Ausgestaltung einer Verarbeitungseinrichtung,

Fig. 7 eine alternative Ausgestaltung des erfindungsgemäßen Medizinproduktes mit einem Drucksensor, der den Hauptsensor bildet,

Fig. 8 eine weitere alternative Ausgestaltung der Erfindung zur Lagebestimmung eines Funktionselementes, sowie

Fig. 9 als Beispiel eines Funktionselementes eine implantierbare Herzpumpe mit einem Rotor.

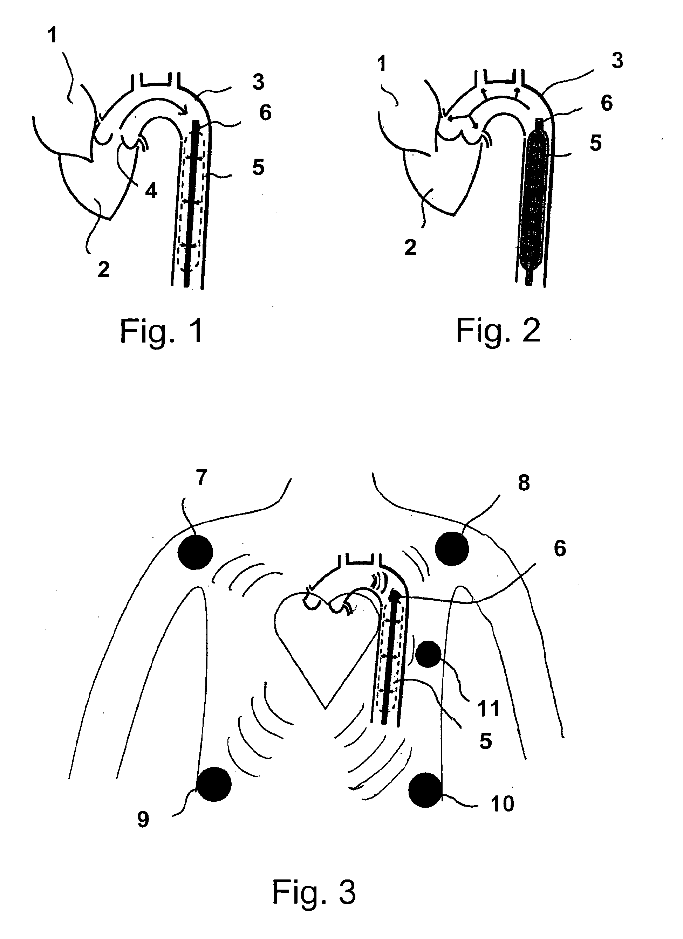

Fig. 1 zeigt schematisch einen Teil eines Herzens mit einem linken Vorhof 1, einem linken Ventrikel 2, von dem ein Blutgefäß 3 ausgeht, und mit Herzklappen 4, die das Blut aus dem linken Ventrikel 2 in das Blutgefäß strömen lassen.

Die Fig. 1 zeigt den Zustand im systolischen Zeitabschnitt, in dem die Ballonpumpe 5 im komprimierten Zustand des Ballons vorliegt. Die Ballonpumpe 5 bildet in diesem Fall das Funktionselement, das zur Unterstützung des Herzens durch Entlastung der linken Herzkammer bei akuter Herzinsuffizienz, koronarer Herzerkrankung und ähnlichen Fällen eingesetzt werden kann. Eine solche intraaortale Ballonpumpe kann einerseits im Rahmen von Koronarbypassoperationen bei der Behandlung des kardiogenen Schocks und akuten Myokardinfarktes sowie auch bei akuter Myokarditis, Kardiomyopathie und akuter Linksherzinsuffizienz eingesetzt werden. Sie wird typischerweise von der Leistenarterie aus in die Aorta vorgeschoben und liegt dann mit der Spitze des Pumpenkatheters am Ende des Aortenbogens. Der Ballon ist typischerweise mit einem Volumen von 40 ml Heliumgas auf einer Länge von 20 cm füllbar und hiermit expandierbar. Der Ballon wird periodisch über eine externe Pneumatik gefüllt und entleert (komprimiert). Durch die entgegengesetzt zur Herztätigkeit verlaufende Ballonfüllung und -entleerung wird während der Systole die Nachlast für die linke Herzkammer gesenkt, was eine Steigerung der Auswurffraktion und eine Senkung des myokardialen Sauerstoffverbrauchs zur Folge hat.

Durch die diastolische Füllung des Pumpballons, dargestellt in Fig. 2, werden im Mittel 40 ml Blut verdrängt, wodurch der diastolische Blutdruck angehoben und in dieser Phase die Organperfusion und insbesondere die Koronarperfusion verbessert wird.

Wichtig ist in diesem Zusammenhang eine möglichst optimale Positionierung des Funktionselementes in der Aorta relativ zur Herzklappe 4 bzw. zur Mündung des Blutgefäßes in das linke Ventrikel.

Die Erfindung bezieht sich in diesem Zusammenhang, wie in Fig. 3 dargestellt, auf einen Hauptsensor 6, der in dem dargestellten Beispiel an der Spitze der Ballonpumpe, jedenfalls aber in festem räumlichem Bezug, beispielsweise in festem, bekanntem Abstand zu dieser oder direkt an dieser, angeordnet ist und der die Erfassung von elektrophysiologischen Signalen des Herzens erlaubt. Hierzu ist der Hauptsensor beispielsweise als Elektrode oder Antenne, je nach interner Beschaltung, ausgebildet.

Zusätzlich sind Hilfssensoren 7, 8, 9, 10, 11 vorgesehen, die durch außen am Körper des Patienten aufgebrachte Elektroden zur EKG-Ableitung gebildet sein können. Es sind ersatzweise auch anders platzierte Sensoren denkbar, beispielsweise Sensoren, die im Rahmen von implantierten Geräten nicht auf der Körperoberfläche, sondern im Körperinneren angeordnet, jedoch zur Aufnahme von elektrophysiologischen Herzsignalen geeignet sind. Solche Sensoren können beispielsweise in Herzschrittmachern oder implantierten Defibrillatoren vorgesehen sein.

Es ist auch das Vorsehen einer geringeren Zahl von Hilfssensoren, beispielsweise ein, zwei, drei oder vier Hilfssensoren denkbar, wobei eine höhere Anzahl die Ausbildung eines komplexeren EKG-Vektors erlaubt, der durch den Vergleich und die Verknüpfung mit den Signalen des Hauptsensors 6 eine genauere Lokalisierung des Hauptsensors und damit des Funktionselements 5 erlaubt.

Sowohl der Hauptsensor 6 als auch die Hilfssensoren nehmen jeweils einen zeitlichen Verlauf der elektrophysiologischen Herzsignale auf, so dass durch Vergleich und Verknüpfung der Signale des Hauptsensors mit Signalen der übrigen, unterschiedlich positionierten Hilfssensoren eine Lagebestimmung des Hauptsensors vorgenommen werden kann.

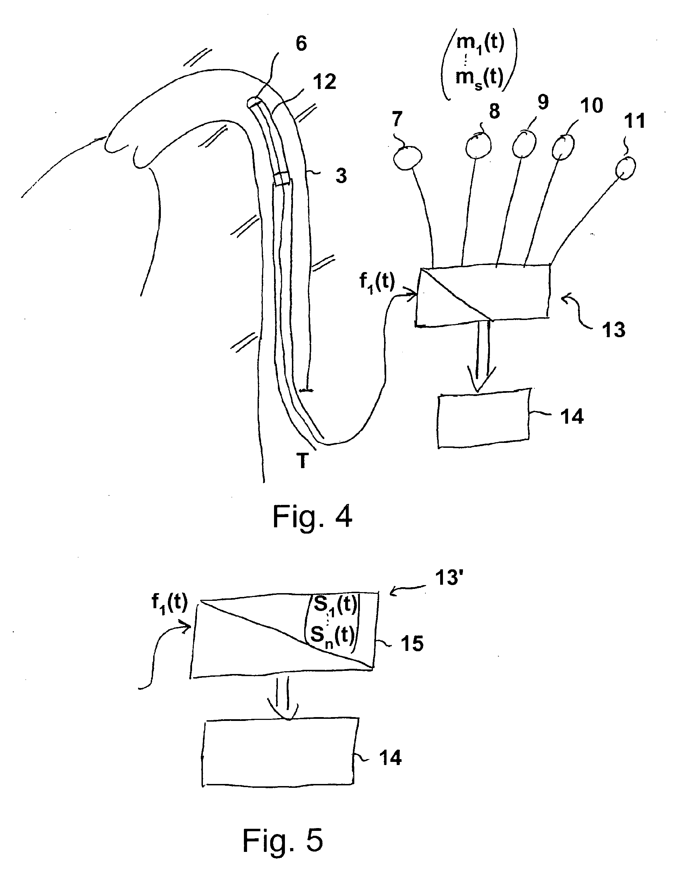

Fig. 4 zeigt hierzu zusätzlich zu dem Funktionselement 12, das beispielsweise auch ein Stent, ein Thrombenfilter, eine Rotationspumpe oder ein Fräskopf sein kann, dem entsprechenden Hauptsensor 6 an der Spitze des Funktionselementes und den Hilfssensoren 7, 8, 9, 10, 11 eine Verarbeitungseinrichtung 13, in der die Signale aller Sensoren zusammengeführt werden.

Die Signale der Hilfssensoren werden zu einem

Vektor $\begin{pmatrix} m_1(t) \\ \vdots \\ m_5(t) \end{pmatrix}$ zusammengefasst, während das

zeitlich veränderliche Signal des Hauptsensors 6 als Skalar $f_1(t)$ vorliegt. Der Vektor der Signale der Hilfssensoren wird mit der Funktion $f_1(t)$ verglichen, um in der Verarbeitungseinrichtung 13 über eine vorgegebene Metrik die Lagebestimmung des Hauptsensors 6 durchzuführen und diese im Ergebnis in der Anzeigevorrichtung 14 anzuzeigen. Dabei kann in der Anzeigevorrichtung entweder der absolute Abstand des Funktionselementes 12 vom Eingang in das linke Ventrikel oder die Entfernung entlang des Blutgefäßes 3 angegeben werden, wenn dessen Gestalt genau bekannt ist.

Fig. 5 zeigt eine alternative Ausgestaltung der Verarbeitungseinrichtung, bei der keine aktuell erfassten elektrophysiologischen Signale von Hilfssensoren erfasst werden, sondern auf gespeicherte Daten

$\begin{pmatrix} s_1(t) \\ \vdots \\ s_n(t) \end{pmatrix}$ zurückgegriffen wird. Diese sind in einer Speichereinrichtung 15 der Verarbeitungseinrichtung 13' hinterlegt und sind beispielsweise zu Beginn der Behandlung bei dem Patienten gemessen und archiviert worden, um später durch Vergleich der aktuell gemessenen Signale des Hauptsensors mit typischen Signalen des Patientenherzens die Lagebestimmung des Funktionselementes zu erlauben.

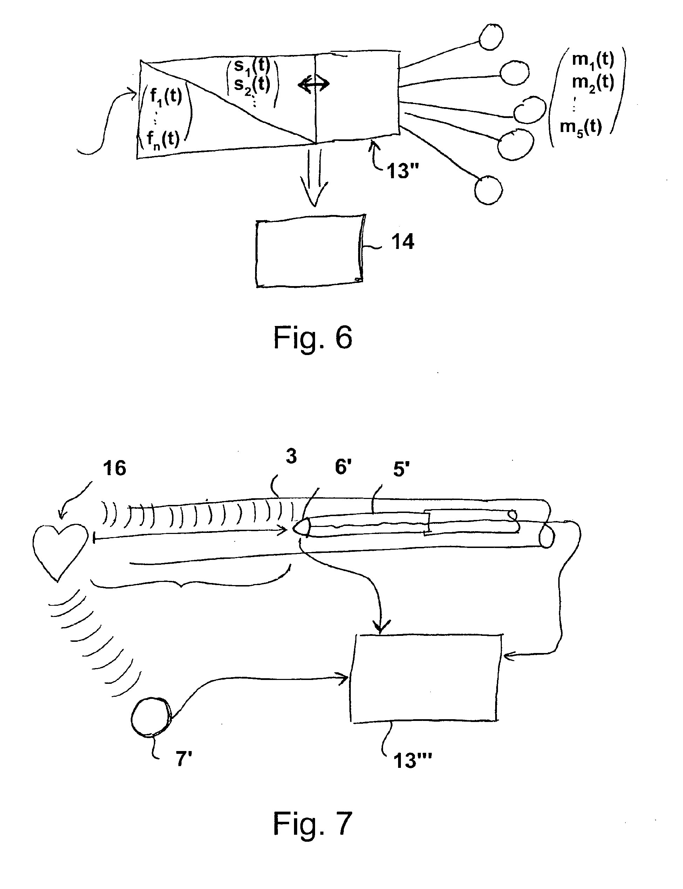

Fig. 6 zeigt eine Variante der Erfindung, bei der sowohl aktuell gemessene elektrophysiologische Herzsignale $\begin{pmatrix} m_1(t) \\ \vdots \\ m_5(t) \end{pmatrix}$ als auch gespeicherte EKG-Daten

$\begin{pmatrix} s_1(t) \\ \vdots \\ s_n(t) \end{pmatrix}$ mit den Signalen eines Hauptsensors verknüpft werden. Dabei können, wie auch in dem anhand von Fig. 4 beschriebenen Beispiel, die Signale des Hauptsensors auch über mehrere an dem Funktionselement 5 räumlich verteilt angeordnete Hauptsensorelemente aufgenommen werden, so dass auch der Hauptsensor die Erfassung eines Signalvektors $\begin{pmatrix} f_1(t) \\ \vdots \\ f_n(t) \end{pmatrix}$

erlaubt. Es können dann zunächst die aktuell erfassten elektrophysiologischen Herzsignale $\begin{pmatrix} m_1(t) \\ \vdots \\ m_5(t) \end{pmatrix}$ mit den

gespeicherten Daten $\begin{pmatrix} s_1(t) \\ \vdots \\ s_n(t) \end{pmatrix}$ verglichen und das Ergebnis dieses Vergleichs mit den durch den Hauptsensor erfassten Daten verknüpft werden, um in der Anzeigeeinrichtung 14 über eine Metrik eine Positionsangabe auszugeben. Dabei kann dort entweder ein absoluter Abstand oder auch eine Ampelanzeige in Form eines grünen Lichts für einen erlaubten Abstandsbereich, eines gelben Lichts für einen kritischen Abstandsbereich und eines roten Lichts für einen verbotenen Abstandsbereich des Funktionselementes von der Herzkammer vorgesehen sein. Die Position kann aber auch alternativ oder ergänzend grafisch dargestellt werden, beispielsweise durch eine Darstellung relativ zur Position des Herzens. Außerdem kann die Position auch mittels eines akustischen Signals dargestellt werden, beispielsweise eines Alarmsignals, welches das Verlassen der vorgesehenen Position oder eine Abstandsänderung zu einem gegebenen Ort anzeigt.

Fig. 7 zeigt eine alternative Gestaltung eines Medizinproduktes gemäß der Erfindung, bei der ein Herz, symbolisch dargestellt und mit 16 bezeichnet, gleichzeitig elektrophysiologische Signale aussendet, die mittels eines oder mehrerer Hilfssensoren 7' registriert werden und infolge der periodischen Pumpaktivität auch Blutdruckschwankungen über das Gefäßsystem transportiert werden, die mittels eines Drucksensors 6' an der Spitze eines Funktionselementes 5' , beispielsweise einer Herzpumpe, registriert werden können.

Da die elektrophysiologischen Signale durch die Sensoren 7' praktisch verzögerungsfrei registriert werden, geben diese eine aktuelles Bild der Herzaktivität, das mit den durch die geringere Wanderungsgeschwindigkeit verzögert bei dem Hauptsensor 6' eintreffenden Druckschwankungen verglichen werden kann. Es kann ermittelt werden bzw. ist bekannt, zu welchen Zeitpunkten bezüglich einer kompletten Herzperiode bestimmte maximale oder minimale Blutdruckwerte im Herzen erzeugt werden, so dass bei bekannter Wanderungsgeschwindigkeit von Druckwellen aus der zeitlichen Differenz der Registrierung durch die Sensoren 6' , 7' auf die Entfernung des Hauptsensors 6' von der Herzkammer geschlossen werden kann. Damit kann mit der dargestellten Konstellation und einer entsprechenden Verarbeitungseinrichtung 13''' eine Lagebestimmung des Hauptsensors 6' und damit des Funktionselementes 5' in Bezug auf das Gefäßsystem im Verhältnis zum Herzen durchgeführt und die Lage angezeigt werden.

Typischerweise wird die Messung anhand des erreichten Druckmaximums durchgeführt. Die Messung mit der Verarbeitungseinrichtung 13''' kann beispielsweise bei Einbringen des Funktionselementes 5' durch langsames Einschieben des Blutgefäßes bei gleichzeitiger bildgebender Lagebestimmung des Funktionselementes 5' geeicht werden.

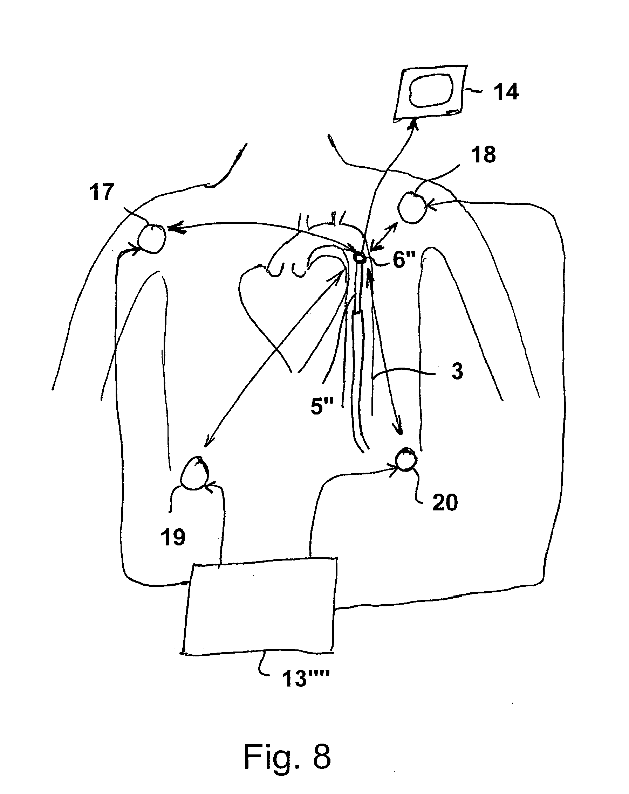

Fig. 8 zeigt eine weitere Alternative des erfindungsgemäßen Medizinprodukts, bei der ein Hauptsensor 6'', der an der Spitze eines Funktionselementes 5'' angeordnet ist, mit mehreren, insbesondere zwei, drei oder vier Hilfssensoren bzw. Transceivern 17, 18, 19, 20 wechselwirkt, beispielsweise über Magnetfelder, elektrische Felder oder elektromagnetische Kopplung. Auch als Hauptsensor 6'' kann ein entsprechender Transceiver eingesetzt werden, so dass entweder dieser Signale sendet, die von den Elementen 17, 18, 19, 20 empfangen werden, oder umgekehrt. Durch die Intensität der Kopplung der Elemente 17, 18, 19, 20 jeweils mit dem Element 6'' kann dessen Position relativ zu den Elementen 17, 18, 19, 20 bestimmt werden. Als Transceiverelemente 17, 18, 19, 20 können auch beispielsweise ohnehin vorhandene Elektroden verwendet werden, die im Wechsel mit der Positionsbestimmung als EKG-Elektroden verwendet werden.

Die Verknüpfung der verschiedenen Kopplungsstärken findet in der Verarbeitungseinrichtung 13'''' statt. Es können auch in diesem Fall an dem Hauptsensor mehrere Sensorelemente angeordnet sein, um beispielsweise die Lagebestimmung genauer zu gestalten oder zusätzlich eine Orientierungsbestimmung des Funktionselementes zu erlauben.

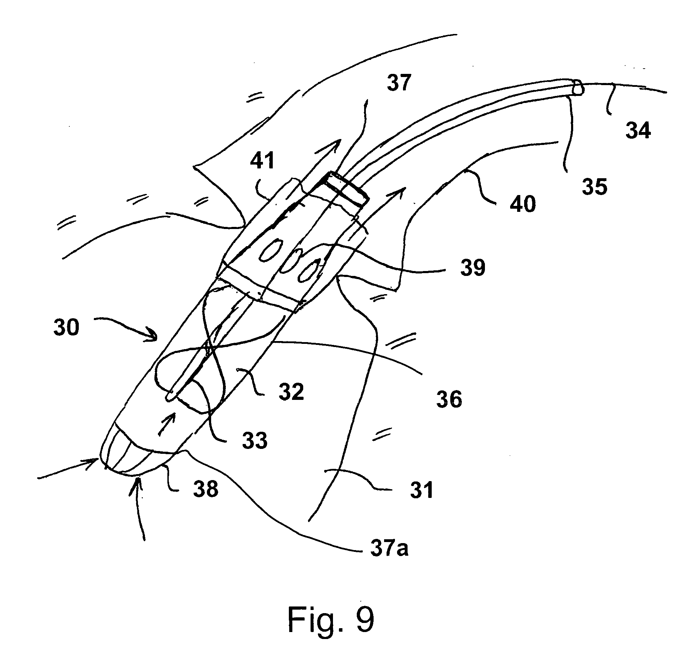

Fig. 9 zeigt beispielhaft als Funktionselement eine Rotationspumpe 30, die zur Einführung in eine Herzkammer 31 komprimierbar und expandierbar gestaltet ist. Die Pumpe 30 weist einen Rotor 32 mit einer Nabe 33 auf, die mittels einer Antriebswelle 34 von außerhalb des Patientenkörpers antreibbar ist. Die Antriebswelle ist durch einen nur teilweise dargestellten Hohlkatheter 35 geführt. Die Pumpe 30 weist ein komprimierbares Pumpengehäuse 36 auf, an dessen proximalem Ende ein Hauptsensor 37 befestigt ist. Der Hauptsensor kann beispielsweise über einen im Lumen oder der Gehäusewand des Hohlkatheters 35 verlaufenden elektrischen Leiter, insbesondere auch über die Antriebswelle, mit einer außerhalb des Körpers angeordneten Verarbeitungseinrichtung verbunden sein.

Die Pumpe saugt im Betrieb Blut über den Ansaugkäfig 38 an und stößt dieses durch Ausstoßöffnungen 39 in das Blutgefäß 40 aus. Es ist ein flexibler Abströmschlauch 41 vorgesehen, der die Ausstoß-/Abströmöffnungen 39 periodisch verschließt und so das Rückströmen von Blut verhindert.

Kann die Position des Hauptsensor 37 bestimmt werden, so kann auch die Pumpe 30 richtig in der Herzkammer positioniert werden.

In einer Abwandlung ist ein weiterer Sensor 37a am distalen Ende des Pumpengehäuses befestigt. In diesem Fall ist es möglich, auch ein entsprechendes Signal, beispielsweise einen EKG-Vektor, direkt zwischen diesen beiden Sensoren zu bestimmen und aus diesem auf die Lage bzw. Lageveränderung der Pumpe zu schließen. Abhängig von den Genauigkeitsanforderungen an die Lagebestimmung kann in diesem Fall eventuell auch auf weitere, insbesondere externe Hilfssensoren verzichtet werden.

## Patentansprüche

1. Medizinprodukt mit einem Funktionselement (5, 5', 5'', 12) zum invasiven Einsatz im Körper eines Patienten und mit einem in festem räumlichem Bezug zu dem Funktionselement stehenden Hauptsensor (6, 6', 6''), **gekennzeichnet durch** eine Verarbeitungseinrichtung (13, 13', 13'', 13''', 13'''') , die Signale des Hauptsensors empfängt und die wenigstens eine die Position des Funktionselementes repräsentierende Größe aus Signalen des Hauptsensors unter Berücksichtigung physiologischer, insbesondere elektrophysiologischer Signale (m(t), s(t)) des Herzens, Hirns oder dergleichen (1, 2, 16) ermittelt.

2. Medizinprodukt nach Anspruch 1, **dadurch gekennzeichnet, dass** die Verarbeitungseinrichtung (13, 13'', 13''') mit mindestens einem, insbesondere zwei, drei, vier oder fünf Hilfssensoren (7, 8, 9, 10, 11) zur Ableitung von elektrophysiologischen Herzsignalen verbindbar ist und aktuell erfasste Signale (m(t)) der Hilfssensoren oder hieraus ermittelte Daten mit den Signalen des Hauptsensors (6, 6', 6'') verknüpft, um eine die Position des Funktionselementes (5, 5', 5'', 12) relativ zum Herzen repräsentierende Größe zu bestimmen.

3. Medizinprodukt nach Anspruch 1 oder 2, **dadurch gekennzeichnet, dass** die Verarbeitungseinrichtung (13', 13'') mit einer Speichereinrichtung (15) versehen ist, in der früher erfasste elektrophysiologische Herzsignale (s(t)), insbesondere des Herzens des Patienten, gespeichert sind, und dass die Verarbeitungseinrichtung zur Ermittlung der die Position des Funktionselementes (5, 5', 5'', 12) repräsentierenden Größe Signale des Hauptsensors (6, 6', 6'') mit gespeicherten Herzsignalen oder hieraus ermittelten Daten verknüpft.

4. Medizinprodukt nach Anspruch 1, 2 oder 3, **dadurch gekennzeichnet, dass** der Hauptsensor (6, 6', 6'') eine Elektrode und/oder Antenne für elektrophysiologische Signale aufweist.

5. Medizinprodukt nach Anspruch 1, 2 oder 3, **dadurch gekennzeichnet, dass** der Hauptsensor (6, 6', 6'') wenigstens zwei voneinander beabstandete Elektroden und/oder Antennen aufweist.

6. Medizinprodukt nach Anspruch 1 oder 2, **dadurch gekennzeichnet, dass** der Hauptsensor (6') einen Sensor zur Erfassung einer strömungsmechanischen Größe, insbesondere einen Drucksensor, aufweist und dass die Verarbeitungseinrichtung (13''') das zeitliche Verhältnis zwischen aktuell erfassten elektrophysiologischen Herzsignalen und einer die Blutströmung an der Position des Hauptsensors charakterisierenden Messgröße erfasst und hieraus eine für die Position des Funktionselementes repräsentative Größe ermittelt.

7. Medizinprodukt nach einem der Ansprüche 1 bis 6, **dadurch gekennzeichnet, dass** das Funktionselement (5, 5', 5'', 12) mittels eines Katheters durch ein Blutgefäß (3) bewegbar ist.

8. Medizinprodukt nach Anspruch 7, **dadurch gekennzeichnet, dass** das Funktionselement eine Blutpumpe, insbesondere eine intraaortale Ballonpumpe, eine Rotationspumpe oder ein Fräskopf, ein Stent oder ein Thrombenfilter ist.

9. Medizinprodukt nach Anspruch 1 oder einem der folgenden, **dadurch gekennzeichnet, dass** die die Position des Funktionselementes repräsentierende Größe der Abstand des Funktionselementes (5, 5', 5'', 12) von der Mündung eines Blutgefäßes (3), in dem sich das Funktionselement befindet, in eine Herzkammer (2), insbesondere entlang dem Verlauf des Blutgefäßes gemessen, ist.

10. Medizinprodukt nach Anspruch 1 oder einem der folgenden, **dadurch gekennzeichnet, dass** der Hauptsensor (6, 6', 6'') unmittelbar an dem Funktionselement (5, 5', 5'', 12) angeordnet ist.

11. Medizinprodukt mit einem Funktionselement zum invasiven Einsatz im Körper eines Patienten und mit einem im festen räumlichen Bezug zu dem Funktionselement stehenden Hauptsensor, **gekennzeichnet durch** wenigstens eine, insbesondere zwei, drei, vier oder mehr als vier außerhalb des Körpers angeordnete Elektroden/Antennen (7, 8, 9, 10, 11, 17, 18, 19, 20) die mit dem Hauptsensor (6, 6', 6'') wechselwirken und **durch** eine Verarbeitungseinrichtung (13''''), die Signale des Hauptsensors und/oder der Elektroden/ Antennen auswertet, um eine die Position des Funktionselementes repräsentierende Größe zu bestimmen.

12. Medizinprodukt gemäß einem der Ansprüche 1 bis 11, **dadurch gekennzeichnet, dass** die Verarbeitungseinrichtung zur Verarbeitung von Signalen eines Impedanzsensors und/oder eines Blutdrucksensors und/oder eines Atmungsaktivitätssensors und/ oder eines Sensors für den Sauerstoffgehalt im Blut eingerichtet ist und/oder dass die Verarbeitungseinrichtung mit einem oder mehreren Sensoren der genannten Sensorarten verbunden ist.

13. Verfahren zum Betrieb eines Funktionselementes (5, 5', 5'', 12) im Körper eines Patienten, insbesondere in einem Blutgefäß (3), **dadurch gekennzeichnet, dass** eine die Position des Funktionselementes repräsentierende Größe dadurch ermittelt wird, dass ein in festem räumlichem Bezug zu dem Funktionselement stehender Hauptsensor (6, 6', 6'') wenigstens einen durch die Herzfunktion des Patienten bestimmten Parameter lokal erfasst und in Signale umwandelt und dass weitere die Herzfunktion charakterisierende Daten oder Signale mit den Signalen des Hauptsensors verknüpft werden und hieraus eine die Position des Funktionselementes repräsentierende Größe ermittelt wird.

Fig. 1

Fig. 2

Fig. 3

$$\begin{pmatrix} m_1(t) \\ \vdots \\ m_s(t) \end{pmatrix}$$

6

12

3

7   8   9   10   11

$f_1(t)$

13

T

14

## Fig. 4

13'

$f_1(t)$

$$\begin{pmatrix} S_1(t) \\ \vdots \\ S_n(t) \end{pmatrix}$$

15

14

## Fig. 5

$$\begin{pmatrix} f_1(t) \\ \vdots \\ f_n(t) \end{pmatrix} \qquad \begin{pmatrix} s_1(t) \\ s_2(t) \\ \vdots \end{pmatrix} \longleftrightarrow$$

$$\begin{pmatrix} m_1(t) \\ m_2(t) \\ \vdots \\ m_5(t) \end{pmatrix}$$

13"

14

## Fig. 6

16     3     6'     5'

7'     13'''

## Fig. 7

Fig. 8

Fig. 9

Europäisches
Patentamt
European
Patent Office
Office européen
des brevets

**EUROPÄISCHER RECHERCHENBERICHT**

Nummer der Anmeldung

EP 11 07 5206

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2011/196248 A1 (GRUNWALD SORIN [FR]) 11. August 2011 (2011-08-11) * Absätze [0014], [0079], [0087], [0095], [0097], [0120], [0213] * * Abbildungen 1,3,4,8 * ----- | 1-13 | INV. A61B5/0402 A61B5/06 |
| X | US 2011/015533 A1 (COX JEREMY B [US] ET AL) 20. Januar 2011 (2011-01-20) * Absätze [0098], [0136], [0137], [0223] * * Abbildungen 1,2,5,9,10,27,74,80 * ----- | 1-13 | |
| X | US 5 713 946 A (BEN-HAIM SHLOMO [IL]) 3. Februar 1998 (1998-02-03) * Spalte 3, Zeile 48 * * Spalte 6, Zeilen 47-51 * * Abbildungen 3,5-7 * ----- | 1-7,9-13 | |

RECHERCHIERTE
SACHGEBIETE (IPC)

A61B

Der vorliegende Recherchenbericht wurde für alle Patentansprüche erstellt

| Recherchenort | Abschlußdatum der Recherche | Prüfer |
|---|---|---|
| Den Haag | 29. Februar 2012 | Worms, Georg |

**ANHANG ZUM EUROPÄISCHEN RECHERCHENBERICHT
ÜBER DIE EUROPÄISCHE PATENTANMELDUNG NR.**

EP 11 07 5206

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

29-02-2012

| Im Recherchenbericht angeführtes Patentdokument | Datum der Veröffentlichung | Mitglied(er) der Patentfamilie | Datum der Veröffentlichung |
|---|---|---|---|
| US 2011196248 A1 | 11-08-2011 | US 2011196248 A1<br>WO 2011097312 A1 | 11-08-2011<br>11-08-2011 |
| US 2011015533 A1 | 20-01-2011 | US 2011015533 A1<br>WO 2011041450 A1 | 20-01-2011<br>07-04-2011 |
| US 5713946 A | 03-02-1998 | US 5694945 A<br>US 5713946 A<br>US 5840025 A | 09-12-1997<br>03-02-1998<br>24-11-1998 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5983126 A
- US 6226546 B1

- US 5391199 A
- US 6892091 B1